# EUROPEAN PATENT APPLICATION

(11) **EP 4 843 553 A1**
(43) Date of publication of application: **25.11.2026**
(21) Application number: 26179752.6
(22) Date of filing: 20.05.2026
(51) Int. Cl.: A61J 3/00

(54) **A BIOLOGICAL FLUID DOSING DEVICE AND METHOD FOR DOSING A BIOLOGICAL FLUID PRODUCT WITH ADDITIVE SOLUTIONS/AGENTS**

(30) Priority: 22.05.2025 US 202563810316 P
(71) Applicant: Fenwal, Inc., Lake Zurich, IL 60047 (US)
(72) Inventor: MADSEN, James Gregory, Lake Zurich, 60047 (US); THOMPSON, Kyle, Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

Systems and methods are provided for dosing a biological fluid product with one or more additive solutions, where the amount of additive solution to be used in doping the biological fluid product is based upon a determined amount of biological fluid product, which is present in the product container, and where a higher concentration level than desired additive solution/agent can be combined with a dilution agent within the biological fluid product dosing device prior to delivering the same to the biological fluid product to be dosed.

## Description

### Field of the Application

The present subject matter relates to systems and methods for processing biological fluids. More particularly, the present subject matter relates to the controlled dosing of biological fluids with one or more additive solutions/agents.

### Background

Pathogen inactivation is a process by which a biological fluid product, such as a blood product, can be treated with one or more drugs, agents, additive solutions, light, and/or treatment methods, which is/are intended to target and inactivate or otherwise neutralize viruses, bacteria, and/or parasites that may be present in the biological fluid. To the extent that an agent or solution is used to treat the biological fluid product, the amount and/or the concentration of the agent or solution being added to the biological fluid product may determine the efficacy of the treatment. Additive solutions or agents administered in fixed amounts and/or concentration levels can result in less effective treatments, where too little of the additive solution or agent is present and/or where the agent or additive solution has a less than desired concentration level, or can sometimes have a deleterious effect on the biological fluid product, where too much of an additive solution or a solution at too high of a concentration level is used. Thus, determining a desired amount of additive solution or agent to be administered to the biological fluid product, it can sometimes be important to know how much biological fluid product that is to be treated, is present, so that an appropriate amount of additive solution/agent to be added can be determined.

Furthermore, in some instances, it may be desirable to store some of these additive solutions or agents at concentration levels that differ from the desired concentration levels when the additive solution/agent is to be administered to the biological fluid product. For example, in some instances, storage of an additive solution/agent at a higher concentration level can help to extend the shelf life of a particular additive solution or treating agent, where higher concentrations can sometimes increase its stability and usability over time. Accordingly, it may be desirable to adjust the concentration level of the additive solution/agent prior to the additive solution being administered to the biological fluid product.

Still further, to the extent that the handling and delivery of the additive solution/agent can be automated in a more closed system, the risk of user exposure to potentially hazardous materials and/or risk of contamination can sometimes be reduced. Additionally, automated processes can sometimes allow for greater consistency, accuracy and repeatability, where the possibility for human error might be better avoided and/or reduced.

Accordingly, it may be desirable to provide a system for the dosing of a biological fluid product with one or more additive solutions/agents, where the dosing is based on an amount of the biological fluid product that is determined as being present before being dosed, and where it may be desirable to alter the concentration level of an additive solution/agent prior to the additive solution/agent being administered to the biological fluid product.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a biological fluid dosing device is provided. The biological fluid dosing device includes a manifold having multiple separately selectable valved ports, and a biological fluid product support, which is adapted for receiving a biological fluid product container including an amount of biological fluid product, the biological fluid product container having an inlet adapted to be coupled to one of the multiple separately selectable valved ports of the manifold. The biological fluid dosing device further including one or more additive solution/agent supports each of which are adapted for receiving a respective source of additive solution/agent, where each source of additive solution/agent has an outlet coupled to a respective one of the multiple separately selectable valved ports of the manifold through which the associated additive solution/agent can be delivered, and a syringe adapted for selectively receiving one or more additive solutions/agents from at least some of the respective sources and delivering the received one or more additive solutions/agents to the biological fluid product container via the manifold. The biological fluid dosing device still further includes a controller configured for determining a desired amount of one or more additive solutions/agents to add to the biological fluid product container, and managing the opening and closing of valves associated with the multiple separately selectable valved ports, to enable at least one of the one or more additive solutions/agents to be received by the syringe and delivered to the biological fluid product container thereby dosing the biological fluid product.

In another aspect, a further biological fluid dosing device is provided. The biological fluid dosing device includes a biological fluid product support, which is adapted for receiving a biological fluid product container including an amount of biological fluid product, the biological fluid product container having an inlet adapted to be coupled to one of multiple separately selectable valved ports of a manifold. The biological fluid dosing device further including one or more additive solution/agent supports each of which is adapted for receiving a respective source of additive solution/agent, where each source of additive solution/agent has an outlet, which is adapted to be coupled to a respective one of the multiple separately selectable valved ports of the manifold through which the associated additive solution/agent can be delivered. The biological fluid dosing device still further including a controller configured for determining a desired amount of one or more additive solutions/agents to add to the biological fluid product container, and managing the opening and closing of each of one or more valves associated with the multiple separately selectable valved ports, to enable at least one of the one or more additive solutions/agents to be received by a syringe and delivered to the biological fluid product container thereby dosing the biological fluid product.

In another aspect, a method for dosing a biological fluid product with one or more additive solutions/agents is provided. The method includes receiving a biological fluid product to be treated and determining a desired amount or dose of each of one or more additive solutions/agents to be included with the received biological fluid product. The desired amount of each of the one or more additive solutions/agents is delivered to the received biological fluid product, thereby producing a dosed biological fluid product including the biological fluid product with the desired amount of each of the one or more additive solutions/agents.

These and other features, and advantages of the present disclosure are evident from the following description of one or more exemplary embodiments, with reference to the accompanying drawings.

### Brief Description of the Drawings

Fig. 1 is a block diagram of a biological fluid dosing device for use in dosing a biological fluid product with one or more additive solutions/agents, in accordance with at least one aspect of the present disclosure;
Fig. 2 is a block diagram of a biological fluid dosing device for use in dosing a biological fluid product with one or more additive solutions, in accordance with at least a further aspect of the present disclosure;
Fig. 3 is a perspective view of an exemplary biological fluid dosing device within which a received biological fluid product can be dosed with one or more additive solutions/agents;
Fig. 4 is a partial plan view of an exemplary manifold, which includes multiple separately selectable valved ports, which can be used as part of the exemplary blood dosing device illustrated in Fig. 3;
Fig. 5 is a chart identifying a plurality of potential stopcock positions for use in connection with the multiple separately selectable valved ports of a manifold, and the corresponding fluid communication via the valved ports associated with each of the stopcock positions;
Figs. 6A-6C are multiple partial schematic views exemplifying the combining of multiple additive solutions/agents within a syringe for subsequent delivery;
Fig. 7 is a perspective view of an exemplary disposable fluid circuit for use with the biological fluid dosing device, in dosing procedures where a single additive solution/agent is used;
Fig. 8 is a perspective view of an exemplary disposable fluid circuit for use with the biological fluid dosing device, in dosing procedures where a pair of additive solutions/agents are used, such as where a higher concentration than desired additive solution/agent is used with an additive solution, which is a dilution agent;
Fig. 9 is a flow diagram of a method for dosing a biological fluid product with one or more additive solutions/agents, in accordance with at least one aspect of the present disclosure;
Fig. 10 is a flow diagram of a method for determining an amount of biological fluid product present in a biological fluid product container, in accordance with at least one aspect of the present disclosure; and
Fig. 11 is a flow diagram of a method for using a higher concentration than desired additive solution/agent with a dilution agent in connection with dosing a biological fluid product.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

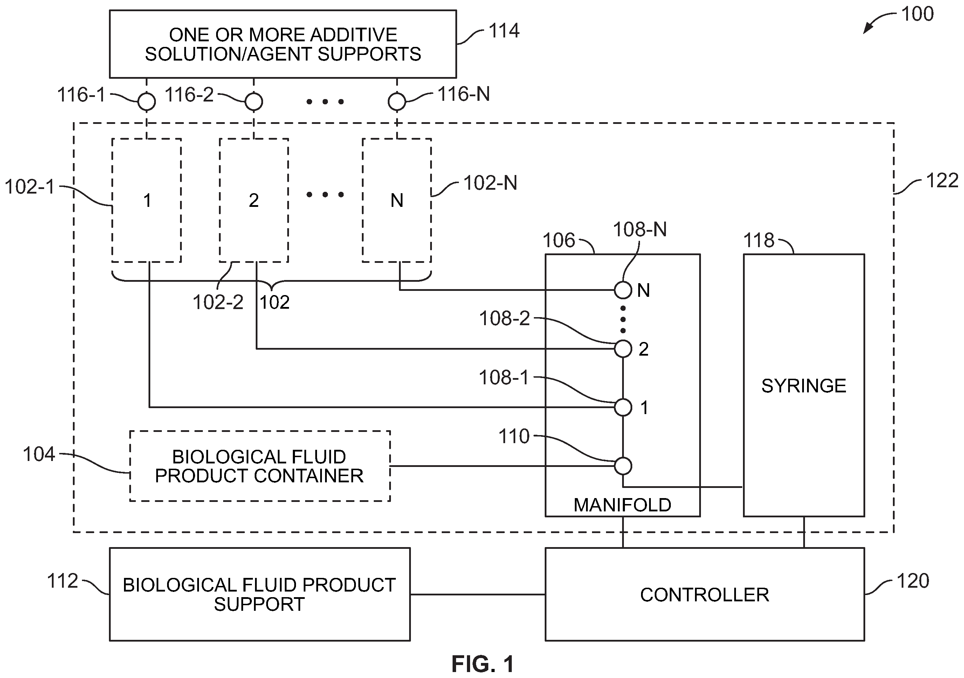

Fig. 1 is a block diagram of a biological fluid dosing device 100 for use in dosing a biological fluid product with one or more additive solutions or agents, in accordance with at least one aspect of the present disclosure. In the illustrated embodiment, the biological fluid dosing device 100 provides for the distribution of fluids between a plurality of containers, which includes one or more sources of additive solutions or agents 102-1, 102-2 and 102-N, and a biological fluid product container 104. The biological fluid dosing device 100 includes a manifold 106 having multiple separately selectable valved ports 108-1, 108-2, 108-N and 110, which allows for the selective delivery of fluids between multiple possible paths. The biological fluid dosing device 100 further includes a biological fluid product support 112, which is adapted for receiving the biological fluid product container 104 having an inlet adapted to be coupled to one of the multiple separately valved ports 110 of the manifold 106.

The biological fluid dosing device 100 still further includes one or more additive solution/agent supports 114. The one or more additive solution/agent supports can include individual supports 116-1, 116-2 and 116-N, which are each adapted for receiving a respective source of additive solution/agent 102-1, 102-2 and 102-N. In some instances the individual supports may merely provide a point of attachment for receiving the respective source of additive solution, such as a hook that can interact with a point of attachment of the source of additive solution/agent 102-1, 102-2 or 102-N, such as a slot that extends through a portion of the additive solution container that is sealed from portion of the container where the associated additive solution/agent is contained. In some instances, the additive solution/agent container can include a plastic pouch that contains the associated additive solution/agent. The respective sources of additive solution/agent generally each have an inlet, which is in fluid communication with the associated cavity, which is coupled to a respective one of the multiple separately selectable valved ports 108-1, 108-2 and 108-N of the manifold 106.

While in some instances, the individual supports can simply provide a point of attachment, in other instances, the individual supports can include other structure or associated devices, that can supply information concerning any associated sources of additive solution/agent. For example, at least some of the individual supports can be associated with a scale, that can be used to determine a weight of the associated source of additive solution/agent, which in turn could be used to estimate an amount of additive solution/agent that is currently contained within the associated source, and/or is leaving the same. Further the individual supports are often elevated relative to the manifold 106 and other portions of the dosing device 100, in order that the contents of any of the sources of additive solution/agent can be gravity fed to the other portions of the dosing device 100.

The biological fluid dosing device 100 additionally includes a pump such as syringe 118, which is adapted for selectively withdrawing one or more additive solutions from at least some of the respective additive solution/agent sources 102-1, 102-2 or 102-N, and delivering the received one or more additive solutions/agents to the biological fluid product container 104 via the manifold 106. While the present disclosure is generally being described whereby the contents of the sources of additive solution/agent are received therefrom by the manifold 106 and are then supplied to the biological fluid product container 104, it is possible that the biological fluid product container 104 can serve as a source of a fluid, which is then conveyed to one or more of the additive solution/agent sources 102-1, 102-2 or 102-N. The syringe 118 can use a created pressure differential to either draw fluids into the syringe 118 or expel the same. By controlling the operational configuration of the separately selectable valved ports of the manifold 106 different ones of the sources of additive solution/agent and the biological fluid product container can be coupled to the syringe 118 as a fluid is respectively being drawn into the syringe 118 or is alternatively being expelled.

The biological fluid dosing device 100 still further includes a controller 120, which is configured for determining a desired amount of one or more additive solutions/agents to add to the biological fluid product container 104, and managing the opening and closing of valves associated with the multiple separately selectable valved ports 108-1, 108-2, 108-N and 110, to enable at least one of the one or more additive solutions/agents to be received by the syringe 118 and delivered to the biological fluid product container 104 thereby dosing the biological fluid product.

In at least some instances, some of the elements associated with the biological fluid dosing device can be part of a disposable fluid circuit 122, configured for one-time use and which can be changed on a per use basis or a limited number of uses. The elements forming the disposable fluid circuit 122 often involve portions of the biological fluid dosing device that can come into contact with the biological fluid product being dosed and/or the dosing agents being added thereto. By changing these elements, cross contamination between dosing procedures can be limited and/or significantly reduced. In at least some embodiments, the disposable fluid circuit 122 can include the biological fluid container 104, the one or more sources of additive solution/agent 102-1, 102-2, 102-N, the manifold 106, and the syringe 118.

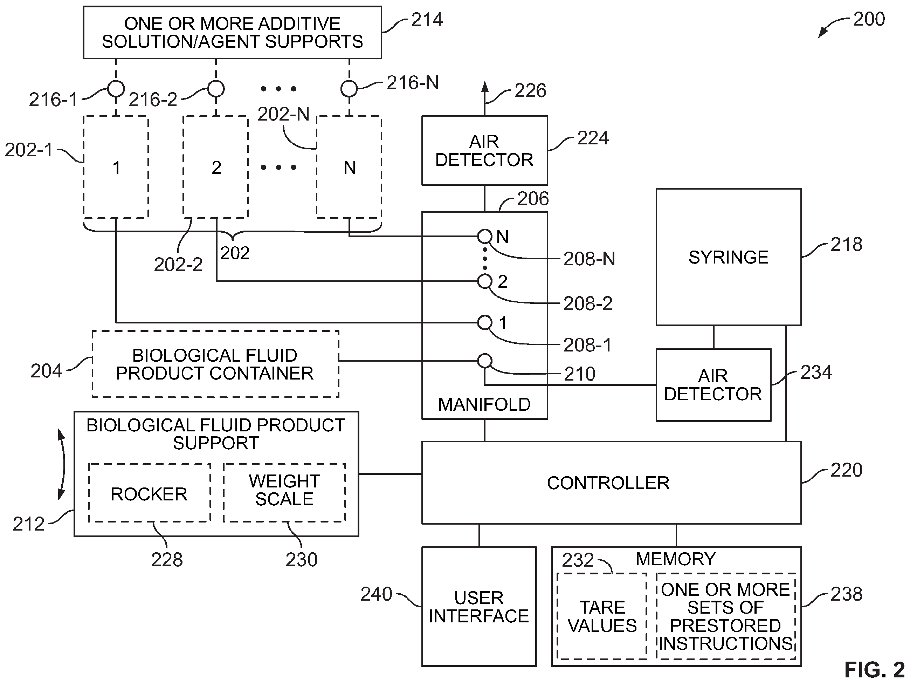

Fig. 2 illustrates a block diagram of a biological fluid dosing device 200 for use in dosing a biological fluid product with one or more additive solutions/agents, in accordance with at least a further aspect of the present disclosure. Similar to the biological fluid dosing device 100 illustrated in Fig. 1, the biological fluid dosing device 200 provides for the distribution of fluids between a plurality of containers, which includes one or more sources of additive solutions/agents 202-1, 202-2 and 202-N, and a biological fluid product container 204. The biological fluid dosing device 200 includes a manifold 206 having multiple separately selectable valved ports 208-1, 208-2, 208-N and 210, which allows for the selective transmission of fluids between multiple possible paths. The biological fluid dosing device 200 further includes a biological fluid product support 212, which is adapted for receiving the biological fluid product container 204 having an inlet adapted to be coupled to one of the multiple separately valved ports 210 of the manifold 206. In the illustrated embodiment, the manifold is further coupled to an air detector 224, which is capable of distinguishing between the presence of liquid or air within a fluid line associated with the manifold, which could be fluidically coupled to the ambient environment 226.

The biological fluid product support, in the illustrated embodiment, includes an agitator 228 and a weight scale 230. The agitator 228 or rocker can be used to facilitate mixing of any fluids present in a biological fluid product container 204 that has been received by the biological fluid product support 212, including instances in which a syringe 218 may be delivering an additive solution/agent to the biological fluid product container 204. The agitator 228 can be used to produce a rocking motion that causes any fluids which are present in a received biological fluid product container 204 to move back and forth within the container 204. The weight scale 230 can be used to determine a weight of the biological fluid container 204 that has been received. Any such weight measurement would generally correspond to the weight of the container 204 including the weight associated with the content of the container. In at least some instances this information may be used to determine an amount of biological fluid product that is present in the biological fluid product container 204. In some instances, the controller 220 will have access to and make use of a tare weight 232, which will be intended to be generally related to the empty weight of the biological fluid product container 204. By better understanding the amount of biological fluid product that is initially present in the biological fluid product container, a determination as to an amount of additive solution/agent to be used as part of any dosing process may be better determined.

The biological fluid dosing device 200, similar to the dosing device 100 described in connection with Fig. 1, still further includes one or more additive solution/agent supports 214, which can include individual supports 216-1, 216-2 and 216-N, which are each adapted for receiving a respective source of additive solution/agent 202-1, 202-2 and 202-N.

The biological fluid dosing device 200, similar to the dosing device 100 described in connection with Fig. 1, additionally includes a syringe 218, which is adapted for selectively withdrawing one or more additive solutions/agents from at least some of the respective additive solution sources 202-1, 202-2 or 202-N, and delivering the received one or more additive solutions/agents to the biological fluid product container 204 via the manifold 206. In the present embodiment, a further air detector 234 may be present between the syringe 218 and the manifold 206. Such an air detector may similarly be able to distinguish between air and a liquid being present in the coupled connection between the syringe 218 and the manifold 206.

The biological fluid dosing device 200, similar to the dosing device 100 of Fig. 1, still further includes a controller 220, which is configured for determining a desired amount of one or more additive solutions/agents to add to the biological fluid product container 204, and managing the opening and closing of valves associated with the multiple separately selectable valved ports 208-1,, 208-2, 208-N and 210, to enable at least one of the one or more additive solutions/agents to be received by the syringe 218 and delivered to the biological fluid product container 204 thereby dosing the biological fluid product, which might be present therein.

The controller 220 may be or be a part of a computer having an operating system, which executes apparatus operation software that may be written in a programming language. Any such software could be stored in a related memory 238, which could be separate from or integrated as part of the controller 220, or could alternatively be stored elsewhere in the biological fluid dosing device 200. The controller 220 may also use hardware to implement the disclosed operations. For example, the controller 220 may be any programmable processor, which could perform the dosing related functions with the aid of one or more sets of instructions 240, which could be stored in the related memory 238. The memory 238 may similarly be used to store any tare values related to the biological fluid product container 204. Any such value could be preloaded or could be received via any input/output functionality related to the controller. An example of at least one such input/output functionality could be related to a user interface 240, which may be associated with the controller 220. An example of a user interface could include a touch screen, keyboard and/or mouse. The user interface could also make use of a microphone associated with voice recognition software and a speaker for supporting audible communication between the user and the controller. Other forms of communication may be supported including connectivity with another device, such as another dosing device, a server, a general purpose or a specific purpose computer, such as a personal computer, a tablet, or a personal communication device, such a cellular telephone.

The disclosed embodiments, including related controllers may also be implemented on a general-purpose or special purpose computer, a programmed microprocessor or microcontroller, a peripheral integrated circuit element, an application-specific integrated circuit or other integrated circuits, hardware/electronic logic circuits, such as a discrete element circuit, a programmable logic device, such as a programmable logic array, field programmable gate-array, or the like. In general, the controller 220, may be any controller or processor device or devices capable of operating an apparatus and implementing the disclosed embodiments.

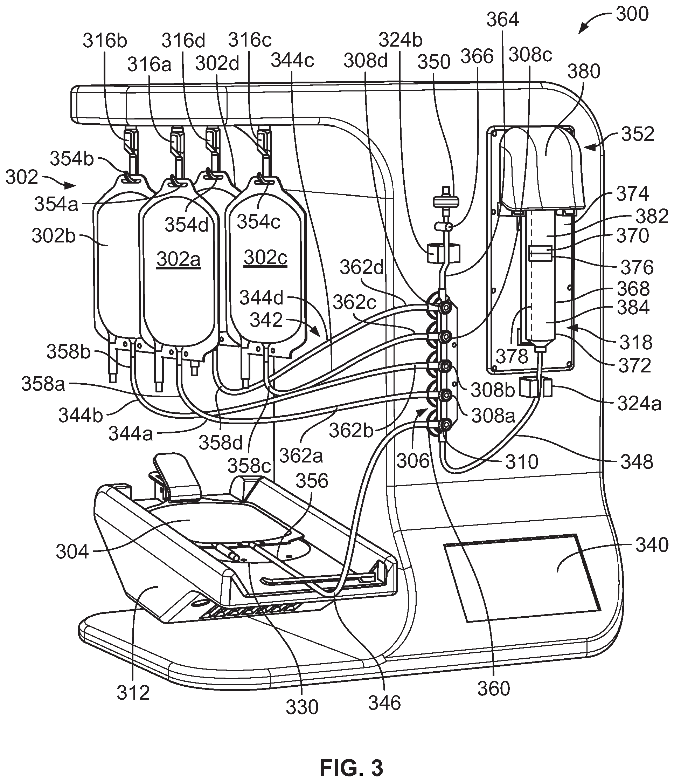

In at least some instances, the biological fluid dosing device can be used to process blood, as described for example in Published International Patent Application No. WO 2024/155598, the contents of which are incorporated herein by reference, as well as described in connection with a corresponding exemplary embodiment which is illustrated in Fig. 3. More specifically, Fig. 3 illustrates a perspective view of an exemplary biological fluid dosing device 300 within which the dosing of a biological fluid product with one or more additive solutions/agents can be implemented.

The biological fluid dosing device 300 is configured as a durable item that is capable of long-term use. It should be understood, that the biological fluid dosing device 300 is merely exemplary of one possible configuration and that the biological fluid dosing devices according to the present disclosure may be configured differently.

In the illustrated embodiment, the biological fluid dosing device 300 has a fluid circuit 342 mounted thereon that includes a manifold 306 having a plurality of separately selectable valved ports implemented in the form of a plurality of stopcocks 310, 308a-d, fluid lines 344a-d, 346, 348, and a vent 350. These components of the fluid circuit 342 may be fluidly connected. The system also includes the plurality of containers 302a-d, 304 that are connectable to the fluid circuit 342 via respective fluid lines 344a-d, 346. The biological fluid dosing device 300 includes a distribution pump 352 for moving/distributing fluid from one of the containers through the fluid circuit 342 and into another container. The biological fluid dosing device 300 further includes air fluid sensors 324a, 324b for detecting liquid and gas (air) within the fluid circuit 342. The biological fluid dosing device 300 also includes a user control interface 340 and a controller, shown in Figs. 1 and 2, for controlling the distribution/movement of fluid through the system 300.

While the embodiment illustrated in Fig. 3 includes five containers 302a-d, 304, the system 100 may alternatively include more or fewer containers, depending on the application. Containers 302a-d, 304 may be sealed containers, such as flexible bags made from a polymeric material. In other embodiments, the containers 302a-d, 304 may be vials, jars, or any other suitable container. In one alternative, the containers may be a mix of different types of containers, wherein the type of each container depends on the particular application or the type of fluid in the container.

The set of containers 302 may include container 302a, 302b, 302c and 302d. In one configuration of the system 300, containers 302a-d are each supply containers and container 304 is a product or pooling container which receives fluid from the set of containers 302. Each of the supply containers 302a-d contains fluid (such as a solution or suspension) that is distributed from the respective container 302a-d and dosed into the product or pooling container 304. In one embodiment, the supply containers 302a-d may be additive solution/agent containers that contain additive solutions/agents, such as but not limited to, saline, Platelet Additive Solution (PAS), Cryoprotectant (i.e., Glycerol, Dimethyl Sulfoxide), Human Serum Albumin (HSA), etc. The product or pooling container 304 may contain a blood product (such as whole blood, red blood cells, platelets, plasma, etc.) or cell solution including, but not limited to mononuclear cells (such as for cell modification or cell culturing).

The system may include support members 316a-d, such as hangers or hooks, that support the containers 302a-d. For example, in the illustrated embodiment, one end of the containers 302a-d includes a point of attachment, such as opening 354a-d for receiving the hangers 316a-d so that the containers 302a-d hang therefrom. In this position and due to the forces of gravity, the fluid pools at the other end of the container. One or more of the hangers 316a-d may be operatively connected to one or more scales, which calculate and/or monitor the weight of the respective container 302a-d. The scales may be used to monitor the volume of fluid exiting and entering the container, as well as to possibly provide a determination of an amount of fluid that is currently present within the container.

The system 300 can also include a tray 312 that holds container 304. The tray 312 may be an agitation tray, which moves, such as using a rocking motion to mix/agitate the fluids in the container 304. Optionally, tray 312 also may be temperature controlled. That is, tray 312 may be configured to heat or cool the contents of container 304, depending on the desired application and fluid contained in container 304. Furthermore, tray 312 may include a scale 330 for weighing and monitoring the weight of container 304. For example, the scale may be used to monitor the volume of fluid entering and exiting container 304, as well as be used to determine an amount and/or estimate thereof, of the fluid that may be present in the container 304.

Each of the containers 302a-d, 304 are connected to and in fluid communication with the fluid lines of the fluid circuit 342. Container 304 is connected to one end 356 of fluid line 346, and containers 302a-d are connected to one end 358a-d of respective fluid lines 344a-d. In one embodiment, the containers are releasably connected to the fluid lines. For example, the containers 302a-d, 304 each have a port that includes a male luer lock which engages a respective female luer lock at the ends 356, 358a-d of fluid lines 344a-d, 346.

In another alternative, one or more of the containers 302a-c, 304 may be permanently connected to the fluid line(s)/system. Such permanent connection may be via a sterile tubing weld, which provides closed-system transfers. In another alternative, some of the containers may be permanently connected to the fluid line(s)/system, while others are releasably connected. For example, the container(s) containing the additive solution/agent may be permanently connected to the fluid line(s), while the container(s) receiving the dosing of the additive solution(s)/agent(s) may be releasably connected to the fluid line(s). After a container(s) has received the selected dosing, the container may be disconnected and a new container(s) may be connected to receive dosing.

In one example, containers 302a-d may be connected to the fluid line(s)/system via bonding, while container 304 may be releasably connected via a luer lock. Such a configuration would allow for supplying a dosing solution into multiple containers 304. Once the system has finished a first dosing container 304 with the additive solutions from containers 302a-d, the first container 304 may be disconnected and a different second container 304 may be connected to the system for dosing. In yet another alternative embodiment, container 304 may include an additive solution/agent that is dosed into one or more of the containers 302. In this alternative, container 304 may be more permanently attached to the fluid line(s)/system. Each of the containers 302a-d may be releasably attached to the fluid line(s)/system. After the additive solution/agent is transferred from container 304 and supplied to the containers 302a-d, the containers 302a-d may be disconnected and new containers 302a-d may be connected for dosing.

The other end 360 of fluid line 346 and the other ends 362a-d of fluid lines 344a-d are connected to and in fluid communication with manifold 306. The manifold includes stopcocks 310, 308a-d for controlling the flow of fluid through the system 300. The stopcocks are motorized and the position of each stopcock is controlled by a motorized stopcock actuator. End 360 of fluid line 346 is in fluid communication with stopcock 310. Ends 362a-d of fluid lines 344a-d are in fluid communication with stopcocks 308a-d. In one alternative embodiment, fluid lines 344a-d, 346 are integrated with respective containers 302a-d, 304, and ends 362a-d, 360 of fluid lines 344a-d, 346 are releasably attached to a respective stopcock 308a-d, 310 with, for example, a luer fitting.

The stopcocks 310, 308a-d have different positions, depending on the desired flow through the fluid circuit 342. In the illustrated embodiment, each of the stopcocks 310, 308a-d has at least three positions. In position A, the stopcock closes off fluid communication to and from the container. In other words, in this position, the stopcock prevents fluid from entering and exiting the container. Also, in position A, the stopcock by-pass is closed. In position B, the stopcock opens fluid communication to and from the container. Fluid may enter or exit the container, depending on the operation of the distribution pump 352. In position B, the by-pass is closed. In position C, the stopcock closes off fluid communication to and from the container and the fluid by-pass is open. The flow of fluids through the system 300 and fluid circuit 342 will be explained in more detail below.

The fluid circuit 342 includes a vent 350 that is in fluid communication with manifold 306. For example, vent 350 may be connected through one of the stopcocks of the manifold 306. In the illustrated embodiment, the vent 350 is in fluid communication with manifold 306 via fluid line 364. Fluid line 364 has one end attached to and is in fluid communication with vent 350 and another end in fluid communication with stopcock 308d. A flow controller 366, such as an in-line pinch valve, may be associated with the fluid line 364. The pinch valve may, optionally, include a sealing head. The flow controller 366 opens and closes the flow of fluid to and the from the vent 350. Vent 350 may include a filter, such as a sterilizing filter. For example, vent 350 may include a 0.2 µm sterilizing filter or any other suitable sterilizing filter. Vent 350 enables, priming of the fluid circuit or lines of the fluid circuit, air-chases, burping of containers, etc.

The system 300 includes a distribution pump 352 that is in fluid communication with the manifold 306. For example, pump 352 may be connected to one of the stopcocks of the manifold 306. In the illustrated embodiment, pump 352 is in fluid communication with manifold 306 via fluid line 348. Fluid line 348 has one end attached to and in fluid communication with pump 352 and another end attached to and in fluid communication with stopcock 310. Pump 352 may be a pneumatic syringe pump, such as the pneumatic syringe pumps disclosed in U.S. Published Application No. 2021/0121827, which is hereby incorporated herein by reference. The distribution pump 352, such as the pneumatic syringe pump, is configured to use a syringe 318 with a syringe barrel 368 (which may be made of cyclic olefin copolymer, or other suitable materials, which may be inert and/or optically clear) and a piston or plunger head assembly 370. The piston head assembly 370 is moveable (translatable) between a first end 372 and a second end 374 of the barrel 368.

The piston head assembly 370 includes the piston and an infrared reflector 376, which defines one part of a position detector. The position detector also includes a plurality of transmitter/sensor pairs 378, which may be in the form of infrared light emitting diodes, and the sensors may be in the form of infrared sensors. According to other embodiments, the transmitters/sensors may also and/or alternatively use visible or ultraviolet light, for example. The transmitter/sensor pairs 378 are disposed along the length of the barrel 368 between the first end 372 and the second end 374. The reflector 376 may be in the form of a reflective strip that is disposed and/or attached opposite the piston.

In operation, the position detector would use the interaction between the transmitter/sensor pairs 378 and the reflector 376 to determine the position of the piston head assembly 370 along the barrel 368. In particular, light emitted from the transmitter would be received by the sensor (or would be received over a threshold amount) if the light contacts the reflector 376. Otherwise, the light would not be received by the sensor (or would not be received below the threshold amount). Depending on the amount of light received by the sensor, a signal generated by the sensor could vary or produce a detectable change. Depending on the signals received from the individual transmitter/sensor pairs, the controller 220 may determine the position of the piston head assembly 370 along the length of the barrel 368 between the first and second ends 372, 374.

A vacuum/pressure source (e.g., a diaphragm pump) 380 is attached to the end 374 of the barrel 368. For example, the vacuum/pressure source may be attached via a fluid line (not shown). The end 374 is otherwise closed, forming a first variable volume space 382 between the closed end 374 of the barrel 368 and the piston head assembly 370. Filtered air may be pumped into and out of the space 382 to cause the piston head assembly 370 to move between the first and second ends 372, 374 of the barrel 368. The movement of the piston head assembly 370 causes a second variable volume space 384 to open between the piston head assembly 370 and the first end 372 to receive a fluid (e.g., an additive solution/agent) into the barrel 368.

In operation, the piston head assembly 370 starts at a first position. The controller 220 causes the vacuum/pressure source 380 to operate and draw vacuum behind the piston head assembly 370 (i.e., in space 382). As a consequence, the piston head assembly 370 moves in the direction of the end 374 (i.e., from the end 372 to the end 374) and draws fluid into the space 384. The controller 220 may subsequently operate the vacuum/pressure source to pump pressurized air into the space 382. This causes the piston head assembly 370 to move in the direction of the end 372 (i.e., from the end 374 to the end 372) and push fluid from the space 384.

The system also includes sensors 324a, 324b that sense and monitor liquid and gas flow through the fluid circuit 342. In one alternative, sensors 324a and 324b may be in-line air bubble detectors, which sense when the fluid in fluid lines 348, 364 has changed from liquid to air or vise-versa. Sensor 324a monitors the liquid/air interface in fluid line 348 through which fluid to and from the distribution pump 352 flows. In one example, when drawing liquid into the syringe 318 from one of the containers 302a-d, 304 the piston head 370 will move up and initially draw air from the fluid line into the syringe 318. This air will be followed by the desired liquid. Upon the liquid reaching the syringe 318, the sensor 324a will detect the interface and the system will record the position of the piston head 370/reflector 376 to use as the zero value for liquid within the syringe 318. This is used to determine the liquid volume within the syringe 318.

Sensor 324a may be used to determine when the liquid volume of the syringe 318 is emptied as the sensor detects the liquid/air interface as fluid flows through and out of syringe 318, and through fluid line 348. Sensor 324b monitors fluid line 364 connected to the vent 350 to prevent the filter of the vent from becoming wet. For example, if sensor 324b senses liquid, it will close flow controller 366 to prevent liquid from flowing to the vent 350. The sensors 324a, 324b send signals to the controller 220 so that the controller can adjust the process as needed or provide a signal to the user.

The system includes user interface 340 which may include a video display and a touch screen, as part of a user interface, to program the dosing/distribution system. The interface 340 sends signals to the controller 220.

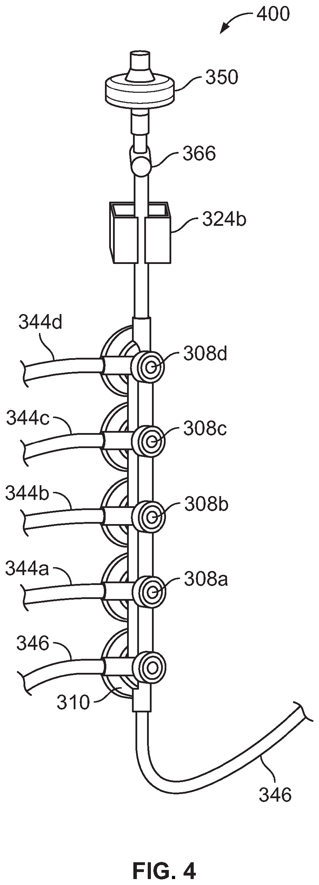

Fig. 4 is a partial plan view 400 of an exemplary manifold, which includes multiple separately selectable valved ports, which can be used as part of the exemplary biological fluid dosing device illustrated in Fig. 3. As noted previously, the exemplary manifold can include the multiple separately selectable valved ports in the form of multiple stopcock 308a-d, 310, which can be separately controlled between different ones of multiple positions to affect the flow of fluids through the manifold, and into the various tubing 344a-d 346, that are each in fluid communication with a respective one of the various fluid containers 302a-d, 304.

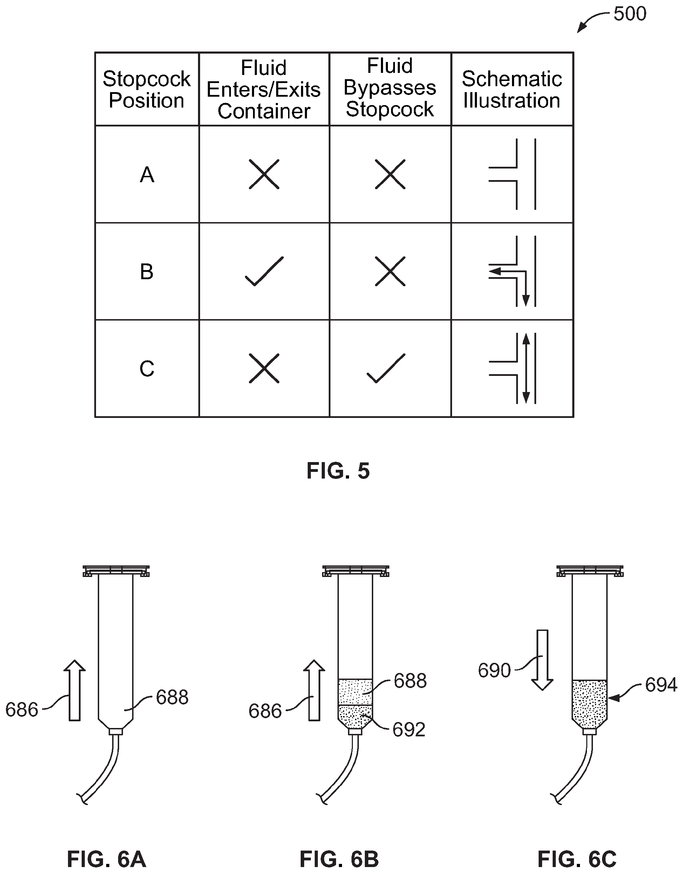

In the illustrated embodiment, each stopcock can have at least three different positions, which can be adjusted under the control of the controller 220, and a respective actuator, to affect the flow of fluids between the various separately selectable valved ports. More specifically Fig. 5 illustrates a chart 500 identifying a plurality of potential stopcock positions for use in connection with the multiple separately selectable valved ports of a manifold, and illustrates the corresponding exemplary fluid communication via the valved ports associated with each of the stopcock positions. In position A, the stopcock closes fluid flow to/from the container and closes the stopcock by-pass. In position B, the stopcock opens fluid flow to/from the container and closes the stopcock by-pass. In position C, the stopcock closes fluid flow to/from the container and opens fluid flow through the by-pass. By managing the position of each of the stopcocks, the flow of fluid between the various fluid containers and the syringe can be managed under the supervision of the controller, where the syringe can be managed to control the respective amount of fluid being drawn into the syringe and then being subsequently expelled.

Figs. 6A-C illustrate the drawing in of a fluid including the potential for drawing in multiple different fluids, before the fluid or combined fluids are then subsequently expelled. More specifically, Fig. 6A illustrates the drawing in 686 of a first fluid 688, where the particular fluid that is being drawn in, is dependent upon the position of each of the stopcocks. This first liquid being present in the syringe could then be expelled 690, into one of the fluid containers dependent upon the configuration of the manifold stopcocks, or further liquid can be drawn into the syringe. This further liquid could be the same or different from the first fluid 688. Fig. 6B illustrates a further second fluid 692 being drawn into 686 the syringe. Where it is desired that the second fluid is different from the first fluid, the positions of each of the stopcocks can be adjusted between each of the respective drawing in of a fluid by the syringe, so that the particular desired liquid from the associated fluid containers can be appropriately selected. The first and second fluids can then be allowed to mix inside the syringe for forming a mixed solution 694 before the same is then expelled 690 to its desired destination.

In such an instance, the mixing of additive solutions or agents within the syringe can allow a first additive solution to be diluted by a second additive solution, such as a saline solution. This would allow the first additive solution/agent to be stored at a higher concentration than might be desired when the additive solution/agent is to be delivered to the biological fluid product. In these or other instances, there may be a value in storing the additive solution or agent at the higher concentration. For example, the additive solution/agent may be more shelf stable at the higher concentration, and/or may have a longer shelf life. Still further, it may be desirable to use the same additive solution or agent for different processes and/or at different times, where alternative varying concentration levels may be preferred at different times relative to the different processes. In these instances, it may be easier to lower the concentration of a particular additive solution or agent through the dilution of the same, than it is to increase the level of concentration. As such a higher starting concentration level may be better able to support the usage of a particular additive solution or agent across a range of possible concentrations and/or a broader range of possible concentrations.

Still further, the potential mixing of different additive solutions/agents prior to using the same to dose a particular biological fluid product, could allow for two relatively less or non-reactive agents to combine, so as to form a third more reactive agent immediately prior to the same being added to the biological fluid product. This may be desirable, where the two less reactive agents may store more easily, and/or may have a longer shelf life.

Just like how a rocker or agitation apparatus can be used as part of the biological fluid product support 112, 212, to better facilitate mixing within the biological fluid product container, an apparatus for agitation, such as a rocker, could similarly be used in conjunction with the syringe to better facilitate the mixing of multiple additive solutions, while present within the syringe.

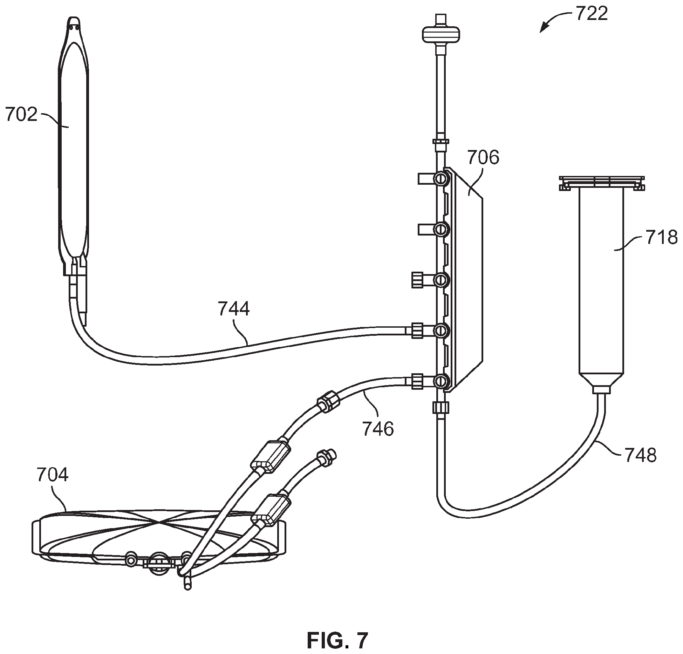

Fig. 7 illustrates a perspective view of an exemplary disposable set 722 for use with the biological fluid dosing device, in dosing procedures where a single additive solution is used. The disposable set 722 would allow for those portions of the dosing device that would come into contact with the biological fluid to be changed out where cross contamination between jobs might be a concern. The disposable set 722 envisions a single additive solution or agent, and might be more applicable in instances where a determination of the amount of biological fluid product that is present in the respective container is desirable for determining a preferred amount of additive to be used as part of any dosing. In such an instance, the disposable set 722 could include at least a single additive solution/agent container 702, which would include the additive solution/agent to be used as part of any dosing, a biological fluid product container 704, a manifold 706, and a syringe 708, as well as any related connections 744, 746, 748, such as tubing, present therebetween.

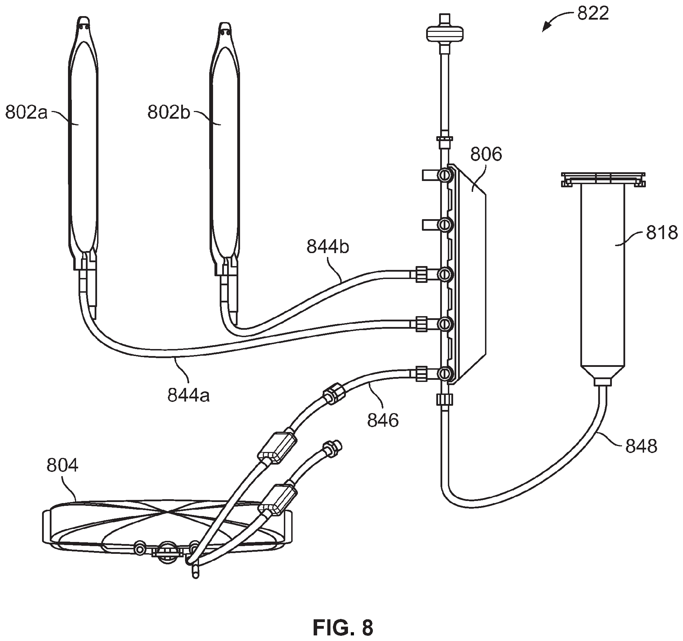

Fig. 8 illustrates a perspective view of an exemplary disposable set 822 for use with the biological fluid dosing device, in dosing procedures where a pair of additive solutions/agents are used, such as where a higher concentration than desired additive solution/agent is used with an additive solution, which is a dilution agent. Similar to Fig. 7, the disposable set can include additive solution/agent containers 802, biological fluid product containers 804, a manifold 806 and a syringe 808, as well as any related connections 844, 846, 848, such as tubing, present therebetween. The primary difference relative to the disposable set for Fig. 7 illustrated in Fig. 7 is the number of additive solutions/agents, where the dilution of a higher concentration than desired additive solution/agent may require at least a pair of additive solutions/agents, which would allow for the second additive/agent solution to be used to dilute the first additive solution/agent. However, while the first disposable set 722 is shown as including a single additive solution/agent and the second disposable set 822 is shown as including a pair of additive solutions/agents, the number of additive solutions/agents to be included with and used as part of any particular disposable set could differ without departing from the teachings of the present application.

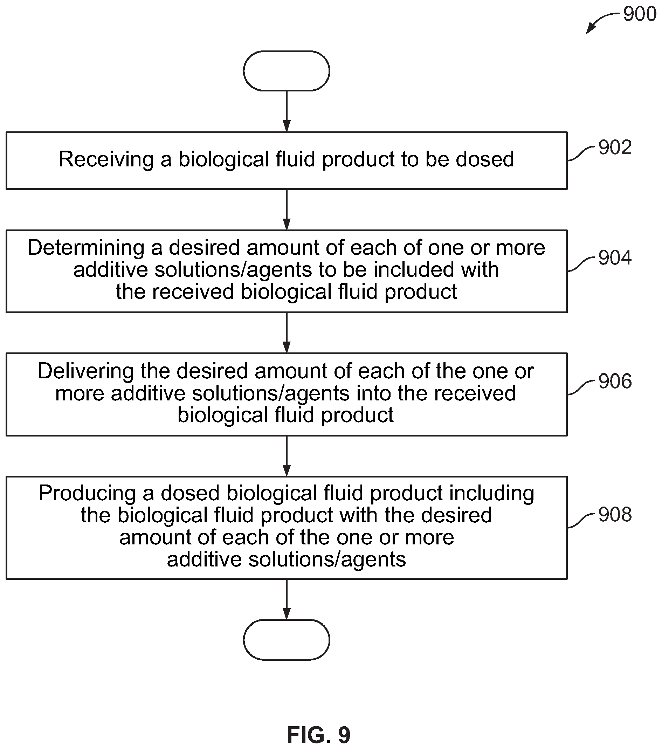

FIG. 9 illustrates a flow diagram of a method 900 for dosing a biological fluid product with one or more additive solutions/agents, in accordance with at least one aspect of the present disclosure. The method includes receiving 902 a biological fluid product to be dosed, and determining 904 a desired amount of each of one or more additive solutions/agents to be included with the received biological fluid product. The desired amount of each of the one or more additive solutions/agents is delivered 906 into the received biological fluid product, thereby producing 908 a dosed biological fluid product including the biological fluid product with the desired amount of each of the one or more additive solutions/agents.

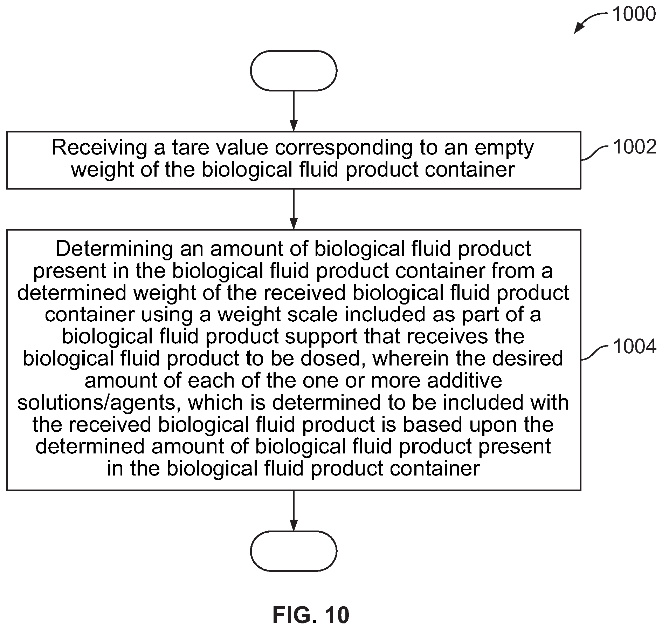

In order to determine the desired amount of each of one or more additive solutions/agents to be included with the received biological fluid product, it may be desirable to determine an amount of biological fluid product, which is present in the biological fluid product container. Fig. 10 illustrates a flow diagram of a method 1000 for determining an amount of biological fluid product that is present in a biological fluid product container, in accordance with at least one aspect of the present disclosure. In the illustrated aspect, the method includes receiving 1002 a tare value corresponding to an empty weight of the biological fluid product container, and determining 1004 an amount of biological fluid product that is present in the biological fluid product container from a determined weight of the received biological fluid product container using a weight scale included as part of a biological fluid product support that receives the biological fluid product to be dosed. The desired amount of each of the one or more additive solutions/agents, which is determined to be included with the received biological fluid product is based upon the amount of biological fluid product, which is determined to be present in the biological fluid product container.

Further, as part of dosing a biological fluid product with an additive solution/agent, it is possible that the additive solution or agent may be at a higher concentration level, than the desired concentration level for the additive solution/agent, when the additive solution/agent is to be delivered to the biological fluid product container. In such an instance it may be possible and desirable to dilute the additive solution/agent at the higher concentration level with a diluting agent, so as to bring the concentration of the additive solution/agent more closely in line with the desired concentration level, prior to the additive solution being added to the biological fluid product present in the biological fluid product container.

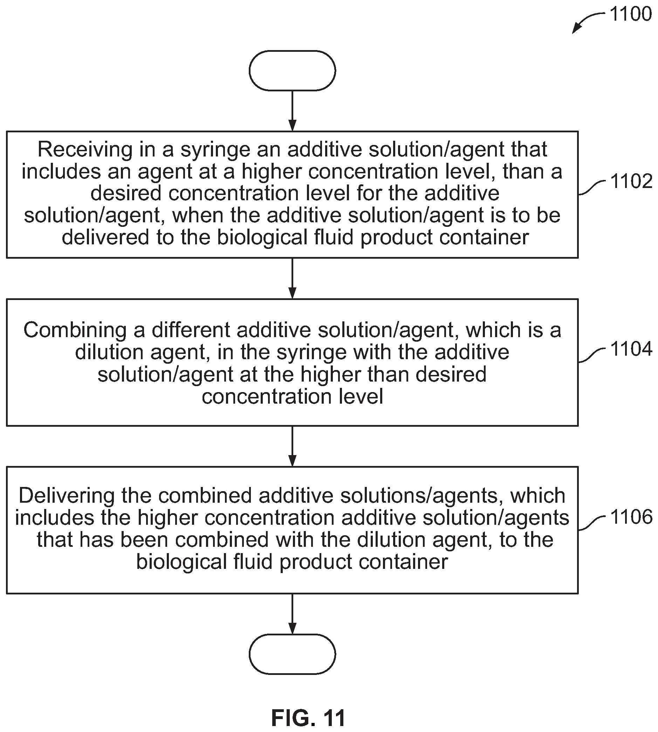

Fig. 11 illustrates a flow diagram of a method 1100 for using a higher concentration than desired additive solution/agent with a dilution agent (diluent) in connection with dosing a biological fluid product. The method includes receiving 1102 in a syringe an additive solution/agent at a higher concentration level, than a desired concentration level for the additive solution/agent, when the additive solution/agent is to be delivered to the biological fluid product container. The method further includes combining 1104 a different additive solution/agent, which is a dilution agent, in the syringe with the additive solution/agent at the higher than desired concentration level, and delivering 1106 the combined additive solutions/agents, which includes the higher concentration additive solution/agent that has been combined with the dilution agent, to the biological fluid product container.

The methods of this disclosure can be implemented on a programmed processor. However, the controllers and flowcharts may also be implemented on a general purpose or special purpose computer, a programmed microprocessor or microcontroller and peripheral integrated circuit elements, an integrated circuit, a hardware electronic or logic circuit such as a discrete element circuit, a programmable logic device, or the like. In general, any device on which resides a finite state machine capable of implementing the flowcharts shown in the figures may be used to implement the controller functions of this disclosure.

Aspects of the present subject matter described above may be beneficial alone or in combination with one or more other aspects. Without limiting the foregoing description, in accordance with one aspect of the subject matter herein, there is provided Aspect 1, a biological fluid dosing device. The biological fluid dosing device includes a manifold having multiple separately selectable valved ports, a biological fluid product support, which is adapted for receiving a biological fluid product container including an amount of biological fluid product, the biological fluid product container having an inlet adapted to be coupled to one of the multiple separately selectable valved ports of the manifold, and one or more additive solution/agent supports each of which are adapted for receiving a respective source of additive solution/agent, where each source of additive solution/agent has an outlet coupled to a respective one of the multiple separately selectable valved ports of the manifold through which the associated additive solution/agent can be delivered. The biological fluid dosing device further includes a syringe adapted for selectively receiving one or more additive solutions/agents from at least some of the respective sources and delivering the received one or more additive solutions/agents to the biological fluid product container via the manifold, and a controller configured for determining a desired amount of one or more additive solutions/agents to add to the biological fluid product container, and managing the opening and closing of valves associated with the multiple separately selectable valved ports, to enable at least one of the one or more additive solutions/agents to be received by the syringe and delivered to the biological fluid product container thereby dosing the biological fluid product.

In accordance with Aspect 2, which may be used or combined with Aspect 1, the biological fluid product support includes a weight scale tray for determining a weight of the received biological fluid product container, and wherein the controller is further configured for determining the amount of biological fluid product present in the biological fluid product container from the determined weight of the received biological fluid product container.

In accordance with Aspect 3, which may be used or combined with Aspect 2, a tare value corresponding to an empty weight of the biological fluid product container is provided to the controller.

In accordance with Aspect 4, which may be used or combined with any one of Aspects 2 and 3, the controller determines the desired amount of each of the one or more additive solutions/agents to be delivered to the biological fluid product container, based upon the amount of biological fluid product determined as being present in the biological fluid product container.

In accordance with Aspect 5, which may be used or combined with any one of Aspects 2 through 4, the amounts of each of the one or more additive solutions/agents being delivered to the biological fluid product container is monitored by a change in the determined weight of the received biological fluid product container by the weight scale tray.

In accordance with Aspect 6, which may be used or combined with any one of Aspects 1 through 5, the amounts of any one of the additive solutions/agents being delivered to the biological fluid product container is determined by the controller in order to produce a selected concentration level of additive solution/agent relative to the contents of the biological fluid product container.

In accordance with Aspect 7, which may be used or combined with any one of Aspects 1 through 6, the one or more additive solution/agent supports include multiple additive solution/agent supports each being adapted for receiving a different additive solution/agent, whose respective outlets are separately coupled to one of the multiple separately selectable valved ports of the manifold.

In accordance with Aspect 8, which may be used or combined with Aspect 7, at least one of the different additive solutions/agents includes a saline solution.

In accordance with Aspect 9, which may be used or combined with any one of Aspects 7 and 8, at least one of the different additive solutions/agents is at a higher concentration level, than a desired concentration for the additive solution/agent, when the additive solution/agent is being delivered to the biological fluid product container.

In accordance with Aspect 10, which may be used or combined with Aspect 9, the additive solution/agent at the higher concentration is received in the syringe and is combined with another one of the different additive solutions/agents, which is a dilution agent, in the syringe, prior to the combined additive solutions/agents being delivered to the biological fluid product container.

In accordance with Aspect 11, which may be used or combined with Aspect 10, the amount of the dilution agent being added is sufficient to bring the concentration levels of the additive solution/agent at the higher concentration level to the desired concentration level, when the additive solution/agent is being delivered to the biological fluid product container.

In accordance with Aspect 12, which may be used or combined with any one of Aspects 1 through 11, the syringe is a pneumatic syringe pump, which facilitates precise volume transfer into and out of the syringe.

In accordance with Aspect 13, which may be used or combined with any one of Aspects 1 through 12, the biological fluid product support includes a rocking mechanism, which is adapted to allow for rocking any biological fluid product container received by the biological fluid product support, as part of the at least one of the one or more additive solutions/agents being delivered to the biological fluid product container.

In accordance with Aspect 14, which may be used or combined with any one of Aspects 1 through 13, the manifold is a stopcock manifold.

In accordance with Aspect 15, which may be used or combined with any one of Aspects 1 through 14, the biological fluid dosing device further includes one or more air detectors for detecting the presence or absence of fluids at different points within the biological fluid dosing device during dosing of the biological fluid product.

In accordance with Aspect 16, which may be used or combined with any one of Aspects 1 through 15, the one or more additive solutions/agents includes a drug which allows for pathogen inactivation in the biological fluid product.

In accordance with Aspect 17, which may be used or combined with any one of Aspects 1 through 16, the one or more additive solutions/agents includes a quenching agent.

In accordance with Aspect 18, which may be used or combined with any one of Aspects 1 through 17, the biological fluid dosing device is a blood dosing device.

In accordance with Aspect 19, which may be used or combined with any one of Aspects 1 through 18, at least some of the respective couplings between the multiple separately selectable valved ports and any received biological fluid containers and any source of additive solution/agent includes tubing.

In accordance with Aspect 20, which may be used or combined with Aspect 19, the biological fluid processing container, the tubing, the manifold, and the syringe are part of a disposable set.

In accordance with Aspect 21, a biological fluid dosing device is provided. The biological fluid dosing device includes a biological fluid product support, which is adapted for receiving a biological fluid product container including an amount of biological fluid product, the biological fluid product container having an inlet adapted to be coupled to one of multiple separately selectable valved ports of a manifold. The biological fluid dosing device further includes one or more additive solution/agent supports each of which is adapted for receiving a respective source of additive solution/agent, where each source of additive solution/agent has an outlet, which is adapted to be coupled to a respective one of the multiple separately selectable valved ports of the manifold through which the associated additive solution/agent can be delivered. The biological fluid dosing device still further includes a controller configured for determining a desired amount of one or more additive solutions/agents to add to the biological fluid product container, and managing the opening and closing of each of one or more valves associated with the multiple separately selectable valved ports, to enable at least one of the one or more additive solutions/agents to be received by a syringe and delivered to the biological fluid product container thereby dosing the biological fluid product.

In accordance with Aspect 22, which may be used or combined with Aspect 21, the biological fluid product support includes a weight scale tray for determining a weight of any received biological fluid product container, and where the controller is further configured for determining the amount of biological fluid product present in the biological fluid product container from the determined weight of the received biological fluid product container.

In accordance with Aspect 23, which may be used or combined with Aspects 22, a tare value corresponding to an empty weight of the biological fluid product container is provided to the controller.

In accordance with Aspect 24, which may be used or combined with any one of Aspects 22 and 23, the controller determines the desired amount of each of the one or more additive solutions/agents to be delivered to the biological fluid product container, based upon the amount of biological fluid product determined as being present in the biological fluid product container.

In accordance with Aspect 25, which may be used or combined with any one of Aspects 22 through 24, the amounts of each of the one or more additive solutions/agents being delivered to the biological fluid product container is monitored by a change in the determined weight of the received biological fluid product container by the weight scale tray.

In accordance with Aspect 26, which may be used or combined with any one of Aspects 21 through 25, the amounts of any one of the additive solutions/agents being delivered to the biological fluid product container is determined by the controller in order to produce a selected concentration level of additive solution/agent relative to the contents of the biological fluid product container.

In accordance with Aspect 27, which may be used or combined with any one of Aspects 21 through 26, the one or more additive solution/agent supports include multiple additive solution/agent supports each being adapted for receiving a different additive solution/agent, whose respective outlets are separately coupled to one of the multiple separately selectable valved ports of the manifold.

In accordance with Aspect 28, which may be used or combined with Aspect 27, at least one of the different additive solutions/agents includes a saline solution.

In accordance with Aspect 29, which may be used or combined with any one of Aspects 27 and 28, at least one of the different additive solutions/agents is at a higher concentration level, than a desired concentration level for the additive solution/agent, when the additive solution is being delivered to the biological fluid product container.

In accordance with Aspect 30, which may be used or combined with Aspect 29, the controller directs the additive solution/agent at the higher concentration level to be received in the syringe and to be combined with another one of the different additive solutions/agents, which is a dilution agent, in the syringe, prior to the controller directing the combined additive solutions/agents in the syringe to be delivered to the biological fluid product container.

In accordance with Aspect 31, which may be used or combined with Aspect 30, the amount of the dilution agent being added is sufficient to bring the concentration levels of the additive solution/agent at the higher concentration level to the desired concentration level, when the additive solution/agent is being delivered to the biological fluid product container.

In accordance with Aspect 32, which may be used or combined with any one of Aspects 21 through 31, the syringe is a pneumatic syringe pump, which facilitates precise volume transfer into and out of the syringe.

In accordance with Aspect 33, which may be used or combined with any one of Aspects 21 through 32, the biological fluid product support includes a rocking mechanism, which is adapted to allow for rocking any biological fluid product container received by the biological fluid product support, as part of the at least one of the one or more additive solutions/agents being delivered to the biological fluid product container.

In accordance with Aspect 34, which may be used or combined with any one of Aspects 21 through 33, the manifold is a stopcock manifold.

In accordance with Aspect 35, which may be used or combined with any one of Aspects 21 through 34, the biological fluid dosing device further includes one or more air detectors for detecting the presence or absence of fluids at different points within the biological fluid dosing device during dosing of the biological fluid product.

In accordance with Aspect 36, which may be used or combined with any one of Aspects 21 through 35, the one or more additive solutions/agents includes a drug which allows for pathogen inactivation in the biological fluid product.

In accordance with Aspect 37, which may be used or combined with any one of Aspects 21 through 36, where the one or more additive solutions/agents includes a quenching agent.

In accordance with Aspect 38, which may be used or combined with any one of Aspects 21 through 37, the biological fluid dosing device is a blood dosing device.

In accordance with Aspect 39, which may be used or combined with any one of Aspects 21 through 38, at least some of the respective couplings between the multiple separately selectable valved ports and any received biological fluid containers and any source of additive solution/agent includes tubing.

In accordance with Aspect 40, which may be used or combined with Aspect 39, the biological fluid processing container, the tubing, the manifold, and the syringe are part of a disposable set, that is adapted to be received by the biological fluid dosing device.

In accordance with Aspect 41, a method for dosing a biological fluid product with one or more additive solutions/agents is provided. The method includes receiving a biological fluid product to be dosed, and determining a desired amount of each of one or more additive solutions/agents to be included with the received biological fluid product. The desired amount of each of the one or more additive solutions/agents is delivered to the received biological fluid product, and a dosed biological fluid product including the biological fluid product with the desired amount of each of the one or more additive solutions/agents is produced.

In accordance with Aspect 42, which may be used or combined with Aspect 41, the method further includes determining an amount of biological fluid product present in the biological fluid product container from a determined weight of the received biological fluid product container using a weight scale included as part of a biological fluid product support that receives the biological fluid product to be dosed, wherein the desired amount of each of the one or more additive solutions/agents, which is determined to be included with the received biological fluid product is based upon the determined amount of biological fluid product present in the biological fluid product container.

In accordance with Aspect 43, which may be used or combined with Aspect 42, determining the amount of biological fluid product present in the biological fluid product container includes receiving a tare value corresponding to an empty weight of the biological fluid product container.

In accordance with Aspect 44, which may be used or combined with any one of Aspects 42 and 43, the method further includes monitoring a change in the determined weight of the received biological fluid product container by the weight scale tray while each of the one or more additive solutions/agents are being delivered to the received biological fluid product, where the amounts of each of the additive solutions/agents being delivered to the biological fluid product container is monitored by a change in the determined weight.

In accordance with Aspect 45, which may be used or combined with any one of Aspects 41 through 44, the amount of any one of the additive solutions/agents being delivered to the biological fluid product container is determined by the controller in order to produce a selected concentration level of additive solution/agent relative to the contents of the biological fluid product container.

In accordance with Aspect 46, which may be used or combined with any one of Aspects 41 through 45, the one or more additive solutions/agents include multiple different additive solutions/agents.

In accordance with Aspect 47, which may be used or combined with Aspect 46, at least one of the different additive solutions/agents includes a saline solution.

In accordance with Aspect 48, which may be used or combined with any one of Aspects 46 and 47, at least one of the different additive solutions/agents is at a higher concentration level, than a desired concentration level for the additive solution/agent, when the additive solution/agent is to be delivered to the biological fluid product container.

In accordance with Aspect 49, which may be used or combined with Aspect 48, the additive solution/agent at the higher concentration level is received in a syringe and is combined with another one of the different additive solutions/agents, which is a dilution agent, in the syringe, prior to the combined additive solutions/agents being delivered to the biological fluid product container.

In accordance with Aspect 50, which may be used or combined with Aspect 49, the amount of the dilution agent being added is sufficient to bring the concentration levels of the additive solution/agent at the higher concentration level to the desired concentration level, when the additive solution/agent is being delivered to the biological fluid product container.

In accordance with Aspect 51, which may be used or combined with any one of Aspects 41 through 50, the method further includes rocking the received biological fluid product, while each of the one or more additive solutions/agents are delivered to the biological fluid product.

In accordance with Aspect 52, which may be used or combined with any one of Aspects 41 through 51, the method further includes detecting the presence or absence of fluids by an air detector at different points within the biological fluid dosing device during dosing of the biological fluid product.

In accordance with Aspect 53, which may be used or combined with any one of Aspects 41 through 52, the one or more additive solutions/agents includes a drug which allows for pathogen inactivation in the biological fluid product.

In accordance with Aspect 54, which may be used or combined with any one of Aspects 41 through 53, the one or more additive solutions/agents includes a quenching agent.

In accordance with Aspect 55, which may be used or combined with any one of Aspects 41 through 54, the biological fluid product is a blood product.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A biological fluid dosing device comprising:
a biological fluid product support, which is adapted for receiving a biological fluid product container including an amount of biological fluid product, the biological fluid product container having an inlet adapted to be coupled to one of multiple separately selectable valved ports of a manifold;
one or more additive solution/agent supports each of which is adapted for receiving a respective source of additive solution/agent, where each source of additive solution/agent has an outlet, which is adapted to be coupled to a respective one of the multiple separately selectable valved ports of the manifold through which the associated additive solution/agent can be delivered; and
a controller configured for determining a desired amount of one or more additive solutions/agents to add to the biological fluid product container, and managing the opening and closing of each of one or more valves associated with the multiple separately selectable valved ports, to enable at least one of the one or more additive solutions/agents to be received by a syringe and delivered to the biological fluid product container thereby dosing the biological fluid product.

2. The biological fluid dosing device of claim 1, wherein the biological fluid product support includes a weight scale tray for determining a weight of any received biological fluid product container, and wherein the controller is further configured for determining the amount of biological fluid product present in the biological fluid product container from the determined weight of the received biological fluid product container.

3. The biological fluid dosing device of any one of claims 1 and 2, wherein the controller determines the desired amount of each of the one or more additive solutions/agents to be delivered to the biological fluid product container, based upon the amount of biological fluid product determined as being present in the biological fluid product container.

4. The biological fluid dosing device of any one of claims 1 through 3, wherein the amounts of each of the one or more additive solutions/agents being delivered to the biological fluid product container is monitored by a change in the determined weight of the received biological fluid product container by the weight scale tray.

5. The biological fluid dosing device of any one of claims 1 through 4, wherein the amounts of any one of the additive solutions/agents being delivered to the biological fluid product container is determined by the controller in order to produce a selected concentration level of additive solution/agent relative to the contents of the biological fluid product container.

6. The biological fluid dosing device of any one of claims 1 through 5, wherein the one or more additive solution/agent supports include multiple additive solution/agent supports each being adapted for receiving a different additive solution/agent, whose respective outlets are separately coupled to one of the multiple separately selectable valved ports of the manifold.

7. The biological fluid dosing device of claim 6, wherein at least one of the different additive solutions/agent is at a higher concentration level, than a desired concentration level for the agent, when the additive solution/agent is being delivered to the biological fluid product container.

8. The biological fluid dosing device of claim 7, wherein the controller directs the additive solution/agent at the higher concentration level to be received in the syringe and to be combined with another one of the different additive solutions/agents, which is a dilution agent, in the syringe, prior to the controller directing the combined additive solutions/agents in the syringe to be delivered to the biological fluid product container.

9. The biological fluid dosing device of any one of claims 1 through 8, wherein the biological fluid dosing device further comprises one or more air detectors for detecting the presence or absence of liquids at different points within the biological fluid dosing device during dosing of the biological fluid product.

10. The biological fluid dosing device of any one of claims 1 through 9, wherein the biological fluid processing container, tubing, the manifold, and the syringe are part of a disposable set, that is adapted to be received by the biological fluid dosing device.

11. A method for dosing a biological fluid product with one or more additive solutions comprising:
receiving a biological fluid product to be dosed;
determining a desired amount of each of one or more additive solutions/agents to be included with the received biological fluid product;
delivering the desired amount of each of the one or more additive solutions/agents into the received biological fluid product; and
producing a dosed biological fluid product including the biological fluid product with the desired amount of each of the one or more additive solutions/agents.

12. The method of claim 11, wherein the amount of any one of the additive solutions/agents being delivered to the biological fluid product container is determined by the controller in order to produce a selected concentration level of additive solution/agent relative to the contents of the biological fluid product container.

13. The method of any one of claims 11 and 12, wherein at least one of the different additive solutions/agent is at a higher concentration level, than a desired concentration level for the additive solution/agent, when the additive solution/agent is to be delivered to the biological fluid product container.

14. The method of claim 13, wherein the additive solution/agent at the higher concentration level is received in a syringe and is combined with another one of the different additive solutions/agents, which is a dilution agent, in the syringe, prior to the combined additive solutions/agents being delivered to the biological fluid product container.

15. The method of any one of claims 11 through 14, wherein the one or more additive solutions/agents includes a drug which allows for pathogen inactivation in the biological fluid product and optionally, a quenching agent.
